# EUROPEAN PATENT SPECIFICATION

(11) **EP 1 089 773 B1**
(45) Date of publication and mention of the grant of the patent: **31.08.2005**
(21) Application number: 99933592.0
(22) Date of filing: 28.06.1999
(51) Int. Cl.: A61L 15/34, A61L 15/48, A61L 15/58, A61F 13/15, D04H 1/64, C09J 9/00, C09J 201/00, C09J 11/00

(54) **HYGIENIC ARTICLE COMPRISING OIL RESISTANT, HYDROPHILIC ADHESIVE**
HYGIENEARTIKEL MIT ÖLRESISTENTEM, HYDROPHILEN KLEBER
ARTICLE D'HYGIENE AVEC UN ADHESIF HYDROPHILE RESISTANT A L'HUILE

(30) Priority: 26.06.1998 EP 98111859; 22.09.1998 EP 98117904
(43) Date of publication of application: 11.04.2001
(73) Proprietor: THE PROCTER & GAMBLE COMPANY, Ohio 45202 (US)
(72) Inventor: LINDNER, Torsten, D-61476 Kronberg (DE); OPPER, Heike, D-65824 Schwalbach (DE); BUSAM, Ludwig, D-65510 Hunstetten (DE); DIVO, Michael, D-61381 Friedrichsdorf (DE); HORN, Thomas, Alexander, D-65929 Frankfurt (DE); MÜLLER, Jörg, D-61184 Karben (DE)
(74) Representative: Hirsch, Uwe Thomas M.H.
(86) International application number: PCT/US1999/014603
(87) International publication number: WO 2000/000229

(56) References cited:
- EP-A- 0 710 737
- WO-A-96/13283
- WO-A-96/16682
- WO-A-97/48779

## Description

### 1. FIELD OF THE INVENTION

This application relates to hygienic articles which are intended to be used in direct contact or in close proximity to the skin of the user. More particularly, the present invention relates to hygienic articles comprising two components which are at least partially joined to each other by means of an oil resistant, hydrophilic adhesive.

### 2. BACKGROUND OF THE INVENTION

Hygienic articles are typically used in direct contact or in close proximity to the skin of the user. Whilst having a wide variety of purposes such as absorption of body exudates (e.g. diapers), covering the skin of the user (e.g. wound dressings), or the like, hygienic articles do face some common performance requirements which result from their exposure to the skin of the user.

Depending on the area of the body they are placed against, hygienic articles must be able to cope with differing amounts of water based liquids. While essentially all regions of the skin perspire and evaporate water, some hygienic articles have to deal with much higher amounts of liquids such as body exudates. To be able to move the liquid away from the skin of the user most hygienic articles comprise at least one liquid pervious component which is placed towards the skin of the user during the intended use.

At the same time, hygienic articles often come into contact with oil-containing compositions. These compositions may be used to protect the skin (e.g. baby creams) or may have been applied to the skin of the user for therapeutic or cosmetic purposes. There also exist hygienic articles in the market which already comprise oil-containing compositions to be transferred to the skin of the user.

However, the oil-containing compositions mentioned above may have detrimental effects on the integrity and the liquid handling properties of the hygienic article. Besides coating the user facing surface of the hygienic article with a hydrophobic (i.e. water repellent) layer, the protective layer can interact with the adhesives comprised in the hygienic article.

Particularly, the adhesive strength of an adhesive may be substantially reduced after the adhesive has been in contact with the oil-containing composition. This potentially causes reduced integrity of the absorbent article, e.g. at least partial separation of components during use. Separation of components then in turn may lead, inter alia, to at least partial interruption of the capillary contact between user facing components and the remaining components of the hygienic article. This finally can substantially reduce the overall liquid handling performance of the hygienic article. Reduced product integrity may also lead to other performance negatives such as complete separation during use, leakage of previously absorbed body exudates, less pleasing product appearance, and the like.

Therefore, it is an object of the present invention to provide an absorbent article which has a sustained product integrity and liquid handling capability during and after contact with oil-containing compositions.

### 3. SUMMARY OF THE INVENTION

It is an object of the present invention to provide a hygienic article comprising a first component being liquid pervious, a second component, and an adhesive whereby at least part of the surface of the first component is joined to at least part of the surface of the second component by means of the adhesive; the hygienic article of the present invention being characterized in that the adhesive has a Contact Angle with distilled water of less than 90° when submitted to the Adhesive Contact Angle Test and a Peel Force Reduction After Oil Application of less than 90%, preferably less than 70%, more preferably less than 50% , even more preferably less than 30%, even more preferable less than 10% when submitted to the Peel Force Reduction Test. Most preferably, the Peel Force Reduction After Lotion Application is negative, i.e. the peel force of the test sample has increased after lotion application.

It is a further object of the present invention to provide a hygienic article wherein the hygienic article further comprises an effective amount of a lotion coating which is partially transferable to the user's skin.

It is a further object of the present invention to provide a hygienic article wherein the hygienic article is a disposable absorbent article. Preferably the first component of the disposable absorbent article is a topsheet, more preferably comprising apertures of at least 0.2mm². Alternatively, the first component of the disposable absorbent article of the present invention is a fecal management member.

Yet another object of the present invention is to provide a disposable absorbent article wherein the second component is an acquisition layer or an absorbent core.

### 4. DETAILED DESCRIPTION OF THE INVENTION

This application relates to hygienic articles comprising two components which are at least partially joined to each other by means of an oil resistant, hydrophilic adhesive.

### 4.1 Hygienic Article

The term "hygienic article" as used herein refers to articles which are intended to be used in contact with or in close proximity to the skin of the user. Hygienic articles include absorbent articles such as absorbent pads, diapers, training pants, adult incontinence devices, sanitary napkins, bed pads, and the like. In addition, hygienic articles comprise articles of low or zero absorbency such as wound dressings, clothing (in particular underwear), and the like. Hygienic articles of the present invention may be intended for multiple use or they may be disposable. The term "disposable" is used herein to describe hygienic articles which are not intended to be laundered or otherwise restored or reused as an hygienic article after a single use.

### 4.2 Oil Resistant, Hydrophilic Adhesive

In order to be able to provide sustained product integrity and fluid handling, the hygienic article of the present invention comprises an oil resistant, hydrophilic adhesive. This adhesive is deployed to at least partially join the surfaces two components of the hygienic article of the present invention to each other. As used herein, the term "joined" encompasses configurations whereby a component is directly secured to another component by affixing the component directly to the other component. The term "partially joined" as used herein refers to configurations in which only parts of the respective surfaces are joined to each other, such as by being joined only at certain points, lines, areas, and the like. Preferably, the adhesive used to join the first and the second component is applied at a basis weight of at least 2 grams per square meter to those interface areas at which the first and the second component are joined to each other. More preferably, the adhesive is applied at a basis weight of at least 4 grams per square meter.

At least the first component of the hygienic article of the present invention must be liquid pervious to allow liquid transfer to at least part of the interface between first component and second component.

An adhesive suitable for the absorbent article of the present invention must satisfy requirements for at least two properties. First, the adhesive must be hydrophilic in order to not negatively affect the liquid penetration into at least the first component. Secondly, the adhesive must be oil resistant in order to maintain at least part of its adhesive strength during or after contact with an oil-containing composition. Generally, the contact of the adhesive with an oil-containing composition may occur on one or more of the following occasions: (i) when the adhesive is applied to a hygienic article already comprising an oil-containing composition (i.e. shortly before applying the adhesive), (ii) when an oil-containing composition is applied to a hygienic article already comprising the adhesive (shortly after applying the adhesive), (iii) during use of the hygienic article e.g. when in contact with skin treated with an oil-containing composition (i.e. long after the application of the adhesive).

An adhesive satisfying the above requirements on oil-resistance and hydrophilicity is available, for example, from Ato Findley Nederlands B.V. of Roosendaal, The Netherlands, under the designation HX9275.

### 4.2.1 Hydrophilicity

The hydrophilicity of an adhesive can be quantified by the Adhesive Contact Angle Measurement described hereinafter. In order to be suitable for the absorbent article of the present invention, the adhesive must have a contact angle with distilled water of less than 90°, preferably less than 80°, more preferably less than 60°, most preferably less than 40°.

### 4.2.1.1 Contact Angle Measurement

The following experimental procedure is used to make adhesive films to be used in measuring the contact angle of the adhesive.
(1) The adhesive is melted. The temperature to which the adhesive is heated should be chosen with care such that the melting procedure has substantially no detrimental effect on the adhesive. A temperature of 160°C has found to be useful for some adhesives.
(2) The adhesive is slot coated onto uncoated paper at a thickness of about 0.125 millimeters (5 mil). As a substrate, the uncoated side of a release paper is used. Such a release paper is available from 4P Rube Göttingen GmbH, of Göttingen, Germany, under the designation of "Rubesil Release Paper HV63-473 (einseitig beschichtet)".

Altemative methods to provide a film of adhesive of substantially uniform thickness of about 0.125 millimeters can also be used. In particular adjustments of the melting temperature have to be made according to the specific adhesive to be tested.

A method and apparatus for determining the contact angle of a droplet of distilled water on a film surface (such as prepared by the procedure outlined above) is described in U.S. patent No. 5,268,733. An apparatus to carry out this measurement is commercially available from TANTEC Inc. of Schaumburg, Illinois, USA, under the designation CONTACT ANGLE METER Model CAM-FILM.

### 4.2.2 Oil resistance

The oil resistance of an adhesive can be quantified by the Peel Force Reduction After Lotion Application Test described hereinafter. In order to be suitable for the absorbent article of the present invention the adhesive must have a Peel Force Reduction After Lotion Application of less than 80%, preferably less than 70%, more preferably less than 50%, even more preferably less than 30%, even more preferably less than 10%. Most preferably, the Peel Force Reduction After Lotion Application is negative, i.e. the peel force of the test sample has increased after lotion application.

### 4.2.2.1 Peel Force Reduction After Lotion Application

The peel force of a two component laminate can be tested using an standard tensile tester such as a Zwick Model 1445, available from Zwick GmbH & Co. of Ulm, Germany, which is interfaced to a Compaq Prolinea 466 computer available from Compaq Computer Corporation of Houston/Texas, USA, using Zwick 7047.4b software which is available from Zwick GmbH & Co. of Ulm, Germany.

The peel force test has to be carried out at ambient conditions (20°C room temperature, 50% relative humidity). The sample to be tested has to be kept at the same ambient conditions for at least 24 hours prior to the test. The tensile tester has to be calibrated and the machine parameters have to be set to a constant crosshead speed of 30.5 centimeters per minute (12 inches per minute). The lower jaw of the tensile tester is positioned to within 20mm from the upper jaw and the displacement is set to zero. The components on one end of the laminate to be tested are peeled apart and then placed into the two jaws of the tensile tester. Finally, the two components of the sample test laminate are pulled apart by the tensile tester recording the respective average peel strength over a measuring distance of at least 40 millimeters.

In order to measure the Peel Force Reduction After Lotion Application, two substantially identical samples of a laminate made from a first component, a second component (both of them being suitable for the present invention), and the adhesive to be tested have to be prepared. In particular, both samples have to be substantially identical in terms of suitable component materials (as discussed below), dimensions, adhesive application pattern, oil-containing composition application pattern (if applicable). The exact type of oil-containing composition application is not critical, as long as it is within the scope as known by the skilled person for application of oil-containing composition in hygienic articles. The peel force of the first sample is measured according to the above peel force test. An oil-containing composition is applied to the outer surface of the first component of the second sample with a basis weight of at least 50 grams per square meter using the following technique. Using a doctor blade, the oil-containing composition is distributed evenly on a polyester film (basis weight 50 grams per square meter). The oil-containing composition coated surface of the film is then placed in direct contact with the outer surface of the first component of the second sample. A suitable oil-containing composition is commercially available from Johnson&Johnson Consumer Products Inc. of Skillman, New Jersey, USA, under the designation of "Johnson's Diaper Rash Ointment". As a replacement for this specific composition an oil-containing composition comprising at least 50% by weight of petrolatum (mineral oil) can be used. Then, the second sample is kept at a temperature of 37°C for 12 hours, the first component being on top of the second component and the coated film being on top of the first component. In addition, a compressive pressure of 5000 Pascal is applied to the film on top of the first component during these 12 hours. Finally, the peel force of the second sample is measured according to the above peel force test. The Peel Force Reduction After Lotion Application is defined as the difference between the peel force of the first sample and the peel force of the second sample, the difference then being normalized to the peel force of the first sample.

### 4.2.3 High Wet Strength Adhesive

In some embodiments of the present invention, it is preferred that the adhesive has a high wet strength. Such adhesives are particularly preferred for usage in combination with fibrous components.

The term "high wet strength adhesive" as used herein refers to an adhesive that exhibits a delayed built-up of the full cohesive strength. For example, such a delayed build-up can be achieved by a delayed crystallization behavior after solidification. The delayed crystallization extends the time during which the adhesive can flow on the surface to which it is applied such as a fiber surface. Surprisingly, it has been found that when used with high surface components such as fibrous components, the use of high wet strength adhesives may lead to improved peel strength especially in the wet state of the components.

The crystallization behavior can be characterized by the measuring viscoelastic properties of the adhesive as characterized by the storage modulus G' as a function of time (see for example: Kulicke, Werner-Michael. Fließverhalten von Stoffen und Stoffgemischen, Hüthig und Wepf, Basel, Heidelberg, New York, 1986, in particular pages 88 through 93). Prior to the measurement, the adhesive is heated above its melting point and subsequently cooled down to the temperature at which the measurement is to be carried out. When the adhesive has reached that temperature, the measurement is started. A detailed description of the method and equipment to measure the storage modulus of an adhesive can be found below.

The time dependence of the crystallization of any hot melt adhesive is characterized by two phases, the initial phase when the adhesive has a low G' and the asymptotic phase when the adhesive has an substantially constant G' (fluctuating less than 5% around its asymptotic value). The initial phase is the time span during which the value of the storage modulus G' is less than 50% of the storage modulus G' in the asymptotic phase.

For purposes of the present invention, an adhesive is considered to be a high wet strength adhesive when the duration of the initial phase is more than 200 seconds, preferably more than 400 seconds, more preferably more than 600 seconds, even more preferably more than 800 seconds, even more preferably more than 1000 seconds, even more preferably more than 1200 seconds, even more preferably more than 1500 seconds, most preferably more than 2000 seconds.

In addition to having a long initial phase, it is preferred when the adhesive has a low value of the storage modulus G' at the beginning of the experiment.

An adhesive which is suitable for the hygienic article of the present invention, i.e. which fulfills the requirement of being a high wet strength adhesive whilst also being lotion resistant and hydrophilic, is available from H. B. Fuller GmbH of Lüneburg, Germany, under the designation D875BD15.

### 4.2.3.1 Storage Modulus Test

With the storage modulus test, the storage modulus G' of an adhesive can be tested, in particular to classify the adhesive as being a high wet strength adhesive.

The storage modulus G' test is carried out in a thermostat chamber at a constant temperature of 60°C +/- 1°C.

Prior to the measurement the adhesive is heated above its melting point. Then the adhesive is inserted into the thermostat chamber. After the adhesive has reached the temperature of the thermostat chamber (60°C), the measurement is started.

The measurement is carried out with a standard plate/cone rheometer. The cone should have an angle of about 4° and the plate should have a diameter of about 40 millimeters. For example, such a rheometer is available under the designation CVO Rheometer System from Bohlin Instruments Inc. of Cranbury, New Jersey, USA, or from Rheometric Scientific of Piscataway, New Jersey, USA, under the designation Dynamic Analyzer RDAII. Typically, the storage modulus is measured with the cone plane rheometer at an oscillation frequency of about 0.5 Hertz.

### 4.2.4 Hot melt adhesives

In order to allow efficient manufacture of the hygienic article of the present invention, hot melt adhesives are preferred for the present invention. The term "hot melt adhesive" as used herein refers to adhesives which are liquefied by heating prior to application, then are applied to the components in the liquid state, and reversibly solidify upon cooling down.

### 4.3 Components

In one embodiment of the hygienic article of the present invention, the first component is a topsheet. The term "topsheet" refers to a component of the hygienic article that is at least partially in direct contact with the skin of the user at least during some time of the intended use. The topsheet is at least partially joined to a second component by means of an oil-resistant, hydrophilic adhesive according to the present invention. This particular embodiment of the hygienic article of the present invention has the advantage that the integrity of the hygienic article after contact with an oil-containing composition is improved compared to conventional hygienic articles. Preferably, the topsheet is at least partially joined to an acquisition/distribution layer as the second component. Equally preferably, the topsheet may be at least partially joined to an absorbent core as the second component. In both cases, the oil resistant, hydrophilic adhesive is used to enhance the liquid transfer from the topsheet to the second component.

In another embodiment of the absorbent article of the present invention the first component is a fecal management member being at least partially joined to second component of the hygienic article. Preferably, the fecal management member is joined to an absorbent core as the second component. Fecal management members are particularly prone to oil-containing composition contact since they are very open to readily accept feces. In addition, they are positioned towards a portion of the user's skin to which oil-containing composition is applied often. Fluid contact between the fecal management member and absorbent core underneath promotes the immobilization of the feces inside the fecal management member by dewatering the feces.

### 4.3.1 Oil-containing composition

In another embodiment of the hygienic article of the present invention, the hygienic article comprises an oil-containing composition which is at least partially transferable to skin of the user. In preferred embodiment of the hygienic article of the present invention, the hygienic article additionally comprises an oil-based composition which is at least partially transferable to the skin of the user during the intended use. Preferably, such an oil-containing composition is positioned on a user facing surface of the hygienic article. The oil-containing composition may also be deployed in such a way that it is only released at the time of intended use such as being microencapsulated.

Preferably, the oil-based compositions suitable for the hygienic article of the present invention have a melting profile such that they are relatively immobile and localized regarding their positioning within the hygienic article at room temperature, are transferable to the user at body temperature, and yet are not completely liquid under extreme storage conditions. Importantly, the oil-based compositions of the present invention are easily transferable to the skin by way of normal contact, user motion, and/or body heat.

The oil-based compositions suitable for the hygienic article of the present invention are solid, or more often semisolid, at 20°C, i.e. at ambient temperatures. By "semisolid" it is meant that the oil-based composition has a rheology typical of pseudoplastic or plastic fluids. When no shear is applied, the oil-based compositions can have the appearance of a semi-solid but can be made to flow as the shear rate is increased. This is due to the fact that, while the oil-based composition contains primarily solid components, it also includes some minor liquid components.

The oil-based compositions suitable for the hygienic article of the present invention are at least semi-solid at room temperature to minimize oil-based composition migration. In addition, the oil-based compositions preferably have a final melting point (100% liquid) above potential "stressful" storage conditions that can be greater than 45°C.

Specifically, the oil-based compositions suitable for the hygienic article of the present invention should have the following melt profile:

| Characteristic | Preferred Range | Most Preferred |
|---|---|---|
| % liquid at | 2-50 | 3-25 |
| room temp. (20 °C) | | |
| % liquid at | 25-95 | 30-90 |
| body temp. (37 °C) | | |
| final melting point (°C) | ≥38 | ≥45 |

By being solid or semisolid at ambient temperatures, these oil-based compositions do not have a tendency to flow and migrate into the interior of the hygienic article to which they are applied. This means less oil-based composition is required for imparting desirable therapeutic or protective coating benefits.

When applied to the user facing surface of hygienic article of the present invention, the oil-based compositions suitable for the hygienic article of the present invention are transferable to the user's skin by normal contact, user motion, and/or body heat.

A preferred embodiment of the hygienic article of the present invention contains an effective amount of an oil-based composition. As used herein, the term "effective amount of an oil-based composition coating" refers to an amount of a particular oil-based composition which, when applied to a diaper topsheet, will be effective in fulfilling their protective, therapeutic, or cosmetic intention. Of course, the effective amount of a oil-based composition coating will depend, to a large extent, on the particular oil-based composition used.

The oil-based compositions suitable for the hygienic article of the present invention comprise: (1) an emollient(s); (2) an immobilizing agent(s) for the emollient; (3) optionally a hydrophilic surfactant(s); and (4) other optional components.

The viscosity of the formulated oil-based compositions, including emollient, immobilizing agent, and optional components should be as high as possible to keep the oil-based composition from flowing into the interior of the hygienic article. Unfortunately, high viscosities can also lead to oil-based compositions that are difficult to apply without processing problems. Therefore, a balance must be achieved so the viscosities are high enough to keep the oil-based compositions localized on the user facing surface of the hygienic article, but not so high as to cause processing problems. Suitable viscosities for the oil-based compositions will typically range from about 5 to about 200 centipoises, preferably from about 15 to about 100 centipoises, measured at 60°C.

### 4.3.1.1 Emollient

The key active ingredient in these oil-based compositions is one or more emollients. As used herein, an emollient is a material that softens, soothes, supples, coats, lubricates, moisturizes, or cleanses the skin. An emollient typically accomplishes several of these objectives such as soothing, moisturizing, and lubricating the skin. For being suitable to be used in the hygienic article of the present invention, these emollients have either a plastic or fluid consistency at 20°C, i.e., at ambient temperatures. This particular emollient consistency allows the oil-based composition to impart a soft, lubricious, lotion-like feel.

Emollients useful in the hygienic article of the present invention can be petroleum-based, fatty acid ester type, alkyl ethoxylate type, fatty acid ester ethoxylates, fatty alcohol type, polysiloxane type, or mixtures of these emollients. Suitable petroleum-based emollients include those hydrocarbons, or mixtures of hydrocarbons, having chain lengths of from 16 to 32 carbon atoms. Petroleum based hydrocarbons having these chain lengths include mineral oil (also known as "liquid petrolatum") and petrolatum (also known as "mineral wax," "petroleum jelly" and "mineral jelly"). Mineral oil usually refers to less viscous mixtures of hydrocarbons having from 16 to 20 carbon atoms. Petrolatum usually refers to more viscous mixtures of hydrocarbons having from 16 to 32 carbon atoms. Petrolatum and mineral oil are particularly preferred emollients for oil-based compositions of the present invention.

### 4.3.1.2 Immobilizing agent(s) for the emollient

The immobilizing agent counteracts the tendency of the emollient to migrate or flow into the hygienic article of the present invention by keeping the emollient primarily localized on the surface of the hygienic article to which the oil-based composition is applied.

Suitable immobilizing agents for the use in the hygienic article of the present invention can comprise a member selected from the group consisting of C₁₄-C₂₂ fatty alcohols, C₁₂-C₂₂ fatty acids, and C₁₂-C₂₂ fatty alcohol ethoxylates having an average degree of ethoxylation ranging from 2 to about 30, and mixtures thereof. Preferred immobilizing agents include C₁₆-C₁₈ fatty alcohols, most preferably selected from the group consisting of cetyl alcohol, stearyl alcohol, and mixtures thereof. Mixtures of cetyl alcohol and stearyl alcohol are particularly preferred. Other preferred immobilizing agents include C₁₆-C₁₈ fatty acids, most preferably selected from the group consisting of palmitic acid, stearic acid, and mixtures thereof. Mixtures of palmitic acid and stearic acid are particularly preferred. Still other preferred immobilizing agents include C₁₆-C₁₈ fatty alcohol ethoxylates having an average degree of ethoxylation ranging from about 5 to about 20. Preferably, the fatty alcohols, fatty acids and fatty alcohols are linear.

### 4.3.1.3 Optional hydrophilic surfactant(s)

It is important that the oil-based composition also be sufficiently wettable to ensure that liquids can rapidly penetrate into at least the first component of the hygienic article. This diminishes the likelihood that body exudates will flow off the oil-based composition coating rather than being drawn into at least the first component. Depending upon the particular immobilizing agent used in the oil-based composition of the present invention, an additional hydrophilic surfactant (or a mixture of hydrophilic surfactants) may, or may not, be required to improve wettability.

### 4.3.1.4 Other optional components

Oil-based compositions can comprise other optional components typically present in emollient, creams, and oil-based compositions of this type. These optional components include water; viscosity modifiers, perfumes, disinfectant antibacterial actives, pharmaceutical actives, film formers, deodorants, opacifiers, astringents, solvents and the like. In addition, stabilizers can be added to enhance the shelf life of the oil-based composition such as cellulose derivatives, proteins and lecithin. All of these materials are well known in the art as additives for such formulations and can be employed in appropriate amounts in the oil-based compositions of the present invention.

### 4.4 Absorbent Article

A particularly preferable embodiment of the hygienic article of the present invention is the disposable absorbent article.

As used herein, the term "absorbent article" refers to devices which absorb and contain body exudates, and more specifically, refers to devices which are placed against the skin of the user to absorb and contain the various exudates discharged from the body of the user. Examples of disposable absorbent articles include feminine hygiene garments such as sanitary napkins and pantiliners, diapers, adult incontinence devices, diaper holders, training pants, and the like.

Disposable absorbent articles typically comprise a liquid pervious topsheet, a liquid impervious backsheet joined to the topsheet and an absorbent core positioned between the topsheet and the backsheet. Disposable absorbent articles and components thereof, including the topsheet, backsheet, absorbent core, and any individual layers of these components, have a body surface and a garment surface. As used herein, "body surface" means that surface of the article or component which is intended to be worn toward or adjacent to the body of the user, while the "garment surface" is on the opposite side and is intended to be worn toward or placed adjacent to the user's body or undergarments when the disposable absorbent article is worn.

The following description generally discusses the absorbent core, topsheet, and backsheet materials that are useful in disposable absorbent articles.

### 4.4.1 Absorbent Core

In general, the absorbent core is capable of absorbing or retaining liquids (e.g., menses, urine, and/or other body exudates). The absorbent core is preferably compressible, conformable, and non-irritating to the user's skin. The absorbent core may be manufactured in a wide variety of sizes and shapes (e.g., rectangular, oval, hourglass, "T" shaped, dog bone, asymmetric, etc.). In addition to the absorbent composites of the present invention, the absorbent core may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these.

The configuration and construction of the absorbent core may also be varied (e.g., the absorbent core may have varying caliper zones and/or have a profile so as to be thicker in the center; hydrophilic gradients; gradients of the absorbent composite of the present invention, superabsorbent gradients; or lower average density and lower average basis weight zones, e.g., acquisition zones; or may comprise one or more layers or structures). The total absorbent capacity of the absorbent core should, however, be compatible with the design loading and the intended use of the absorbent article. Further, the size and absorbent capacity of the absorbent core may be varied to accommodate different uses such as diapers, incontinence pads, pantiliners, regular sanitary napkins, and overnight sanitary napkins, and to accommodate users ranging from infants to adults.

The absorbent core can include other absorbent components that are often used in absorbent articles, for example, a dusting layer, a wicking or acquisition layer, or a secondary topsheet for increasing the user's comfort.

### 4.4.2 Topsheet

The topsheet is preferably compliant, soft feeling, and non-irritating to the user's skin. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

### 4.4.3 Backsheet

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as a film-coated nonwoven material. A suitable backsheet is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-1401 and by Tredegar Film Products of Terre Haute, Indiana, under the designation XP-39385. The backsheet is preferably embossed and/or matte finished to provide a more clothlike appearance. Further, the backsheet may permit vapors to escape from the absorbent core (i.e., the backsheet is breathable) while still preventing exudates from passing through the backsheet. The size of the backsheet is dictated by the size of the absorbent core and the exact absorbent article design selected.

### 4.4.4 Construction

The backsheet and the topsheet are positioned adjacent the garment surface and the body surface, respectively, of the absorbent core. The absorbent core is preferably joined with the topsheet, the backsheet, or both in any manner as is known by attachment means (not shown in Figure 3) such as those well known in the art. However, embodiments of the present invention are envisioned wherein portions of the entire absorbent core are unattached to either the topsheet, the backsheet, or both.

For example, the backsheet and/or the topsheet may be secured to the absorbent core or to each other by a uniform continuous layer of adhesive, a patterned layer of adhesive, or an array of separate lines, spirals, or spots of adhesive. The attachment means will preferably comprise an open pattern network of filaments of adhesive as is disclosed in U.S. Patent 4,573,986, issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zwieker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

### 4.4.5 Other Preferable Embodiments

Another preferable embodiment of the absorbent article of the present invention are diapers. As used herein, the term "diaper" refers to an absorbent article generally worn by infants, and incontinent persons that is worn about the lower torso of the user. In other words, the term "diaper" includes infant diapers, training pants, adult incontinence devices, etc.

Diapers of the present invention can have a number of well known configurations, with the absorbent cores thereof being adapted to the present invention. Exemplary configurations are described generally in U.S. Patent 3,860,003 issued to Buell on January 14, 1975; U.S. Patent 5,151,092 issued to Buell et al. on September 29, 1992; U.S. Patent 5,221,274 issued to Buell et al. on June 22, 1993.

Another preferable embodiment of the absorbent article of the present invention are training pants. The term "training pants", as used herein, refers to disposable garments having fixed sides and leg openings. Training pants are placed in position on the user by inserting the user's legs into the leg openings and sliding the training pant into position about the user's lower torso. Suitable training pants are disclosed in U.S. Patent No. 5,246,433, issued to Hasse, et al. on September 21, 1993.

Another preferable embodiment of the absorbent article of the present invention are incontinence articles. The term "incontinence article" refers to pads, undergarments (pads held in place by a suspension system of same type, such as a belt, or the like), inserts for absorbent articles, capacity boosters for absorbent articles, briefs, bed pads, and the like regardless of whether they are worn by adults or other incontinent persons. Suitable incontinence articles are disclosed in U.S. Patent No. 4,253,461 issued to Strickland, et al. on March 3, 1981; U.S. Patent Nos. 4,597,760 and 4,597,761 issued to Buell; the above-mentioned U.S. Patent No. 4,704,115; U.S. Patent No. 4,909,802 issued to Ahr, et al.; U.S. Patent No. 4,964,860 issued to Gipson, et al. on October 23, 1990; and in U.S. Patent Application Serial No. 07/637,090 filed by Noel, et al. on January 3, 1991 (PCT Publication No. WO 92/11830 published on July 23, 1992).

## Claims

1. A hygienic article comprising
- a first component being liquid pervious,
- a second component,
- an adhesive,
at least part of the surface of said first component being joined to at least part of the surface of said second component by means of said adhesive **characterized in that** said adhesive has
- a Contact Angle with distilled water of less than 90° when submitted to the Adhesive Contact Angle Test and
- a Peel Force Reduction After Oil Impact of less than 90% when submitted to the Peel Force Reduction Test.

2. An hygienic article according to claim 1, wherein said adhesive has a Peel Force Reduction After Oil Impact of less than 70% when submitted to the Peel Force Reduction Test.

3. A hygienic article according to claim 2, wherein said adhesive has a Peel Force Reduction After Oil Impact of less than 50% when submitted to the Peel Force Reduction Test.

4. A hygienic article according to claim 1, wherein said hygienic article further comprises an effective amount of a oil-based composition which is partially transferable to the wearer's skin.

5. A hygienic article according to any of the preceding claims, wherein said hygienic article is a disposable absorbent article.

6. A disposable absorbent article according to claim 5, wherein said first component is a topsheet.

7. A disposable absorbent article according to claim 6, wherein said second component is an acquisition layer or an absorbent core.

8. A disposable absorbent article according to claim 6, wherein said topsheet comprises apertures of at least 0.2mm².

9. A disposable absorbent article according to claim 5, wherein said first component is a fecal management member.

10. A disposable absorbent article according to claim 9, wherein said second component is an absorbent core.

11. A disposable absorbent article according to claim 1, wherein said adhesive is a high wet strength adhesive.

## Patentansprüche

1. Hygieneartikel, umfassend
- eine erste Komponente, die flüssigkeitsdurchlässig ist,
- eine zweite Komponente,
- ein Haftmittel,
wobei mindestens ein Teil der Oberfläche der ersten Komponente mit mindestens einem Teil der Oberfläche der zweiten Komponente mittels des Haftmittels verbunden ist, **dadurch gekennzeichnet, dass** das Haftmittel Folgendes aufweist:
- einen Berührungswinkel mit destilliertem Wasser von weniger als 90 °, wenn es dem Haftmittel-Berührungswinkeltest ausgesetzt wird, und
- nach Öleinwirkung eine Schälkraftreduktion von weniger als 90 %, wenn es dem Schälkraftreduktionstest ausgesetzt wird.

2. Hygieneartikel nach Anspruch 1, wobei das Haftmittel nach Öleinwirkung eine Schälkraftreduktion von weniger als 70 % hat, wenn es dem Schälkraftreduktionstest ausgesetzt wird.

3. Hygieneartikel nach Anspruch 2, wobei das Haftmittel nach Öleinwirkung eine Schälkraftreduktion von weniger als 50 % hat, wenn es dem Schälkraftreduktionstest ausgesetzt wird.

4. Hygieneartikel nach Anspruch 1, wobei der Hygieneartikel weiterhin eine wirksame Menge einer ölbasierten Zusammensetzung umfasst, die teilweise auf die Haut des Trägers übertragbar ist.

5. Hygieneartikel nach einem der vorstehenden Ansprüche, wobei der Hygieneartikel ein Einwegabsorptionsartikel ist.

6. Einwegabsorptionsartikel nach Anspruch 5, wobei die erste Komponente eine Decklage ist.

7. Einwegabsorptionsartikel nach Anspruch 6, wobei die zweite Komponente eine Erfassungsschicht oder ein Absorptionskern ist.

8. Einwegabsorptionsartikel nach Anspruch 6, wobei die Decklage Öffnungen von mindestens 0,2 mm² umfasst.

9. Einwegabsorptionsartikel nach Anspruch 5, wobei die erste Komponente ein Fäkalienhandhabungsteil ist.

10. Einwegabsorptionsartikel nach Anspruch 9, wobei die zweite Komponente ein Absorptionskern ist.

11. Einwegabsorptionsartikel nach Anspruch 1, wobei das Haftmittel ein Haftmittel mit hoher Nassfestigkeit ist.

## Revendications

1. Article sanitaire comprenant
- un premier composant perméable aux liquides,
- un second composant,
- un adhésif,
au moins une partie de la surface dudit premier composant étant liée à au moins une partie de la surface dudit second composant au moyen dudit adhésif **caractérisé en ce que** ledit adhésif a
- un angle de contact avec l'eau distillée inférieur à 90° lorsqu'il est soumis à l'essai d'angle de contact des adhésifs et
- une réduction de la force de pelage après impact d'huile inférieure à 90 % lorsqu'il est soumis à l'essai de réduction de la force de pelage.

2. Article sanitaire selon la revendication 1, dans lequel ledit adhésif a une réduction de force de pelage après impact d'huile inférieure à 70 % lorsqu'il est soumis à l'essai de réduction de la force de pelage.

3. Article sanitaire selon la revendication 2, dans lequel ledit adhésif a une réduction de force de pelage après impact d'huile inférieure à 50 % lorsqu'il est soumis à l'essai de réduction de la force de pelage.

4. Article sanitaire selon la revendication 1, dans lequel ledit article sanitaire comprend en outre une quantité efficace d'une composition à base d'huile qui est partiellement transférable sur la peau de l'utilisateur.

5. Article sanitaire selon l'une quelconque des revendications précédentes, dans lequel ledit article sanitaire est un article absorbant jetable.

6. Article absorbant jetable selon la revendication 5, dans lequel ledit premier composant est une feuille supérieure.

7. Article absorbant jetable selon la revendication 6, dans lequel ledit second composant est une couche de récupération ou un centre absorbant.

8. Article absorbant jetable selon la revendication 6, dans lequel ladite feuille supérieure comprend des orifices d'au moins 0,2 mm².

9. Article absorbant jetable selon la revendication 5, dans lequel ledit premier composant est un élément de gestion des matières fécales.

10. Article absorbant jetable selon la revendication 9, dans lequel ledit second composant est un centre absorbant.

11. Article absorbant jetable selon la revendication 1, dans lequel ledit adhésif est un adhésif de résistance à l'état humide élevée.
